(19) Europäisches Patentamt
European Patent Office
Office européen des brevets

(11) Veröffentlichungsnummer: **0 004 287**
**B1**

(12) # EUROPÄISCHE PATENTSCHRIFT

(21) Anmeldenummer: **79100530.9**

(22) Anmeldetag: **23.02.79**

(51) Int. Cl.³: **C 07 D 207/452,**
C 08 F 220/48,
C 08 F 220/40,
B 01 D 13/04

(54) **Polymerisierbare Maleinimidgruppen enthaltende Disulfimidderivate, Verfahren zu ihrer Herstellung und ihre Verwendung.**

(30) Priorität: **07.03.78 DE 2809713**
**07.03.78 DE 2809696**

(43) Veröffentlichungstag der Anmeldung:
**03.10.79 Patentblatt 79/20**

(45) Bekanntmachung des Hinweises auf die Patenterteilung:
**25.03.81 Patentblatt 81/12**

(84) Benannte Vertragsstaaten:
**DE**

(56) Entgegenhaltungen:
**DE - A - 1 545 834**
**FR - A - 2 132 351**
**US - A - 2 686 774**

(73) Patentinhaber: **BAYER AG**
**Zentralbereich Patente, Marken und Lizenzen**
**D-5090 Leverkusen 1, Bayerwerk (DE)**

(72) Erfinder: **Elfert, Klaus, Dr.**
**Breslauer Strasse 31**
**D-4150 Krefeld (DE)**

(72) Erfinder: **Rosenkranz, Hans Jürgen, Dr.**
**Heinrich-Kauert-Weg 9**
**D-4150 Krefeld (DE)**

(72) Erfinder: **Ritter, Helmut, Dr.**
**Bodelschwinghstrasse 16**
**D-4150 Krefeld 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Polymerisierbare Maleinimidgruppen enthaltende Disulfimidderivate, Verfahren zu ihrer Herstellung und ihre Verwendung

Die Erfindung betrifft neue Maleinimido- und Disulfimidgruppen (=Disulfonylamingruppen) enthaltende ungesättigte Monomere, ein Verfahren zu ihrer Herstellung, sowie ihre Verwendung zur Herstellung von insbesondere zur Ultrafiltration und Umkehrosmose geeigneten semipermeablen Membranen.

Bei einer Vielzahl von synthetischen Polymerprodukten können gewisse Eigenschaftsbilder dadurch erreicht bzw. verbessert werden, daß stark saure Komponenten als Comonomere in die Polymerkette eingebaut werden. Hervorzuheben sind Eigenschaften wie Anfärbbarkeit mit basischen Triphenylmethan-Farbstoffen, antielektrostatisches Verfahren, die Hautverträglichkeit bei Synthesefasern oder das Glasfaser-bzw. Pigmentbenetzungsvermögen. Obwohl eine breite Palette polarer Monomerer wie z.B. Acrylsäure, Maleinsäure, Acrylamid oder Hydroxyäthylacrylat zur Verfügung steht, genügen die durch chemischen Einbau dieser Monomerbausteine erzielten Effekte häufig nicht den gestellten Anforderungen, so daß auf stark polare Systeme, bevorzugt mit sauren Komponenten, zurückgegriffen werden muß.

Comonomere, die stark saure Gruppen in radikalisch polymerisierte Kunststoffe einbringen, stehen bisher jedoch nur in geringer Zahl zur Verfügung. Es sind im wesentlichen nur Methallylsulfonsäure oder Vinylsulfonsäure technisch interessant, wobei noch gewisse Nachteile in Kauf genommen werden. So zeichnet sich Methallylsulfonsäure besonders nachteilig durch die hohe Übertragungskonstante aus, d.h. der Einbau dieses Monomeren in Kunststoffe ist mengenmäßig begrenzt, außerdem wird die Bruttoreaktionsgeschwindigkeit verringert und die Molekulargewichte der resultierenden Polymeren entsprechend erniedrigt. Vinylsulfonsäure kann aufgrund der Instabilität nur in wäßriger Lösung gehandhabt werden, wodurch zusätzliche Kosten, z.B. bei Transport oder Lagerung, entstehen.

Darüberhinaus ist es aus der britischen Patentschrift 867 006 u.a. bekannt, (Meth)Acryloylaminobenzol-benzol-disulfimide durch Umsetzung der Halogenide der (Meth) Acrylsäure mit Aminobenzol-benzol-disulfimiden herzustellen und als Comonomere bei dier radikalischen Polymerisation einzusetzen. Aus der allgemeinen Formel auf Seite 1 dieser Schrift läßt sich durch mosaikartiges Zusammensetzen auch 3-Carboxy(meth)acryloylaminoaryl-aryl-disulfimid (= (Methyl)Maleinsäuremonoamidoaryl-aryl-disulfamid) der Formel.

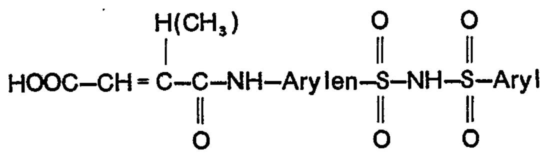

konstruieren, das als Ausgangsmaterial für die Herstellung der erfindungsgemäßen Monomeren eingesetzt bzw. als Zwischenprodukt gebildet wird. Aus den allgemein gehaltenen Angaben der britischen Patentschrift auf Seite 2, Zeilen 34 bis 41 wird zur Herstellung solcher Verbindungen die Umsetzung von Maleinsäureanhydrid mit primäre Aminogruppen enthaltenden Disulfimiden empfohlen. Nähere Angaben über Umsetzungsbedingungen bzw. praktische Beispiele können der britischen Patentschrift nicht entnommen werden. Führt man die Umsetzung von Maleinsäureanhydrid mit Aminobenzol-benzol-disulfimiden nach üblichen Verfahren beispielsweise gemäß US-Patentschrift 3 018 290, Spalte 5, Zeilen 42—47 durch, d.h. in geeigneten Lösungsmitteln in etwa äquimolaren Mengenverhältnissen der Reaktionspartner, so erhält man die N-substituierten Maleinamidsäuren nicht nur in schlechten Ausbeuten, sondern auch stark verunreinigt.

Es ist ferner bekannt, N-substituierte Maleinamidsäuren durch eine Cyclodehydratisierung in organischen Lösungsmitteln bei 0—100°C in Gegenwart von Essigsäureanhydrid und Katalysatoren wie Nickelsalzen und tertiären Aminen (vergl. deutsche Offenlegungsschrift 2 040 094) oder in Anhydriden niedriger Fettsäuren wie Essigsäureanhydrid bei Temperaturen über 25°C in Gegenwart von Katalysatoren wie tertiären Aminen (vergl. US—PS 3 018 290 und 3 018 292) in die entsprechenden Maleinimidderivate zu überführen. N-substituierte Maleinimide, in denen der N-Substituent ein Diarylsulfimid ist, sind jedoch noch nicht bekanntgeworden.

Gegenstand der vorliegenden Erfindung sind nun Verbindungen der allgemeinen Formel (I)

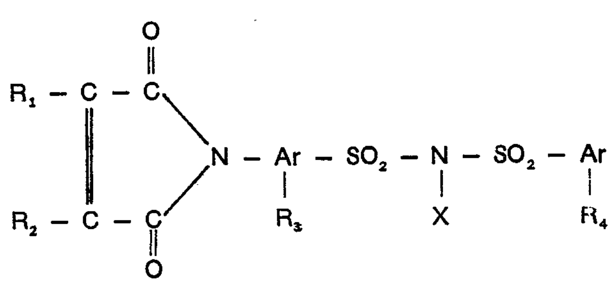

(I)

in der

Ar einen Benzol- oder Naphthalinrest, vorzugsweise einen Benzolrest;

$R_1$ und $R_2$ gleich oder verschieden voneinander H oder $CH_3$;

$R_3$ und $R_4$ gleich oder verschieden voneinander H, Phenyl, Alkyl verzweigt oder linear mit 1—6 C-Atomen, Hydroxyalkyl verzweigt oder linear mit 1—6 C-Atomen oder Cl, F, vorzugsweise H und

X, H, Na, K, $NH_4$, vorzugsweise H oder Na darstellen.

Gegenstand der Erfindung ist auch ein Verfahren zur Herstellung der erfindungsgemäßen N-substituierten Maleinimidderivate durch Umsetzung von Maleinsäureanhydrid oder einem durch $CH_3$ substituierten Maleinsäureanhydrid mit einem entsprechenden Aminoaryl-aryl-disulfimid und anschließender Cyclodehydratisierung der gebildeten N-substituierten Maleinamidsäure in einem Anhydrid einer gesättigten Fettsäure mit maximal 5 C-Atomen in Gegenwart eines Katalysators, dadurch gekennzeichnet, daß die Umsetzung des Maleinsäureanhydrids oder methyl substituierten Maleinsäureanhydrids mit dem Aminoaryl-aryl-disulfimid in der Schmelze in Abwesenheit oder in Gegenwart eines Katalysators erfolgt und das Maleinsäureanhydrid gegenüber dem Aminoaryl-aryl-disulfimid in einem 1 bis 5fachen molaren Überschuß eingesetzt wird.

Das Verfahren verläuft nach folgendem Reaktionschema, wobei die einzelnen Symbole die für Formel (I) angegebene Bedeutung besitzen.

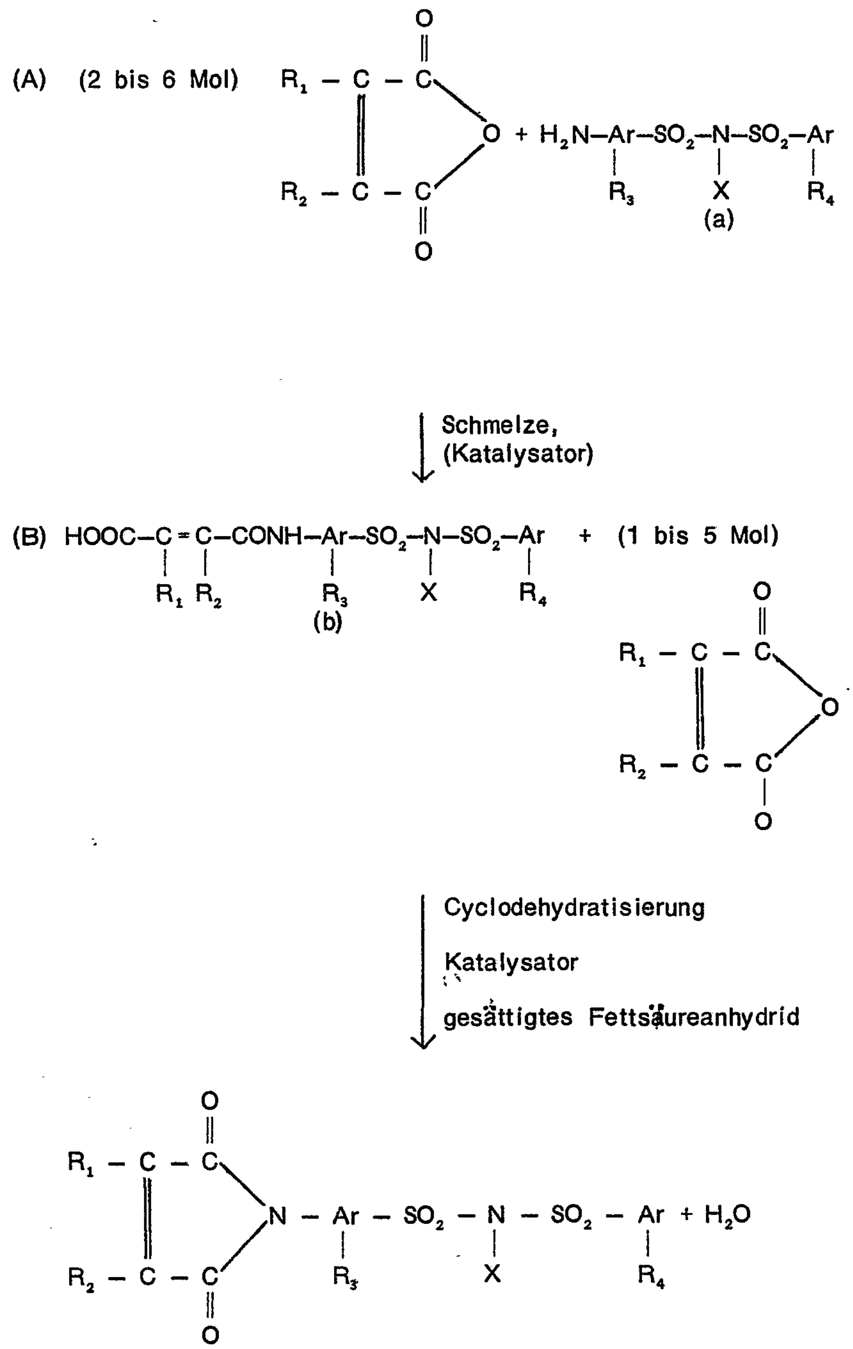

Die als Ausgangssubstanzen für die Reaktion (A) (siehe Reaktionsschema) einzusetzenden Aminoaryl-aryl-disulfimide (a) sind literaturbekannt (vergl. Methoden der Organischen Chemie, Houben-Weyl, Band 11/1 (1957) S. 368) bzw. werden nach bekannten Methoden hergestellt (vergl. deutsche Patentschrift 757 262, deutsche Auslegeschrift 1 249 259, US—PS 2 374 934). Die Umsetzung mit den gegebenenfalls substituierten Maleinsäureanhydriden (auch Citraconsäureanhydrid kann eingesotzt werden) erfolgt in der Schmelze, d.h. bei Temperaturen von etwa 60 bis 180°C, vorzugsweise 100 bis 140°C, wobei auf 1 Mol des Disulfimids 2 bis 6 Mol. vorzugsweise 3 bis 5 Mol, Anhydrid zum Einsatz gelangen. Der Überschuß an Anhydrid kann anschließend leicht durch Destillation im Vakuum oder durch Extraktion mit einem geeigneten Lösungsmittel entfernt werden.

Als Katalysatoren bei der Reaktion (A) können tertiäre Amine der Formel

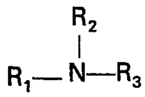

eingesetzt werden, wobei $R_1$, $R_2$ und $R_3$ gleich oder verschieden sein können und Alkylgruppen mit 1—4 C-Atomen bedeuten. Ferner können cyclische tertiäre Amine wie Pyridin, N—$C_1$—$C_4$-alkylierte Morpholine, Piperidine, Piperazine und Triäthylendiamin (Dabco) verwendet werden.

Die vorgenannten Katalysatoren werden vorzugsweise in Mengen von 0,2 bis 2 Gew.-%, bezogen auf eingesetztes ggf. substituiertes Maleinsäureanhydrid, eingesetzt.

Die Cyclodehydratisierung der Maleinsäuremonoamidoaryl-aryl-disulfimide (b) kann nach bekannten Verfahren, beispielsweise den Verfahren der US-Patentschriften 3 018 290 und 3 018 292 oder der deutschen Offenlegungsschrift 2 040 094 erfolgen. Vorzugsweise wird sie gemäß dem Verfahren der US—PS 2 444 536 durchgeführt. d.h. in einem Anhydrid gesättigter Fettsäuren mit 2 bis 5 C-Atomen wie Essigsäureanhydrid in Gegenwart von Alkalisalzen der vorstehenden Fettsäuren mit 2 bis 5 C-Atomen, wie Natriumacetat bei Temperaturen von etwa 25°C bis unterhalb des Zersetzungspunktes der Reaktanten bzw. des gebildeten Maleinamidsäure-disulfimids, insbesondere bei 60—100°C. Das Anhydrid der Fettsäure mit 2 bis 5 C-Atomen wird vorzugsweise in der 1—10fachen molaren Menge, insbesondere 5—8fachen molaren Menge, bezogen auf 1 Mol Maleinamidsäuredisulfimid eingesetzt. Die zugesetzten Mengen an Alkalisalz der genannten $C_2$—$C_5$-Fettsäuren, insbesondere Natriumacetat, betragen vorzugsweise 5—20 Gew.-% der eingesetzten Anhydridmenge des Fettsäureanhydrids. Der Überschuß an eingesetztem Anhydrid sowie gebildete Fettsäure können nach der Cyclodehydratisierung gegebenenfalls im Vakuum abdestilliert werden und das Maleinimidodisulfimid nach Kristallisation isoliert oder aber vor dem Abdestillieren nach Zugabe eines geeigneten Fällungsmittels wie niederen Alkoholen ($C_1$—$C_4$), Wasser (pH=7), Aceton usw. erhalten werden.

Die erfindungsgemäßen Maleinimido-disulfimide sind gut radikalisch copolymerisierbar, ferner als Feststoffpulver einfach zu handhaben und zu lagern und zeichnen sich darüber hinaus durch besonders einfache präparative Zugänglichkeit aus.

Die erfindungsgemäßen Monomeren können radikalisch polymerisiert werden, wobei die Copolymerisation mit Monomeren, wie (Meth)-Acrylsäure, (Meth)-Acrylamid, (Meth)-Acrylsäureester mit 1—12 C-Atomen, vorzugsweise 1—4 C-Atomen in der Alkoholkomponente, Butadien, Styrol, Maleinsäure, Fumarsäure, Vinylchlorid, Vinylidenchlorid, Vinylacetat, Maleinimiden, Vinylpyrrolidon, Vinylcarbazol, Vinylpyridin, Vinylcaprolactam oder Acrylnitril eine besonders bevorzugte Variante ist.

Aus den Copolymeren mit Acrylnitril können bei einem Anteil der erfindungsgemäßen Verbindungen von vorzugsweise 1—5 Gew.-% Fasern hergestellt werden, die sich gegenüber bekannten Polyacrylnitril-Produkten durch verbesserte Anfärbbarkeit mit basischen Farbstoffen auszeichnen und darüber hinaus eine verbesserte Hautverträglichkeit aufweisen.

Prinzipiell war die Copolymerisierbarkeit der neuen erfindungsgemaßen Monomeren mit anderen Monomeren aufgrund der Lehre der US-PS 2.686.774 zu erwarten, und auch die verbesserte Anfärbbarkeit von Fasern aus Copolymerisaten des Acrylnitrils und erfindungsgemäßen Monomeren war im Hinblick auf die GB—PS 867.006, in der diese Eigenschaft von ähnlichen Acrylnitrilcopolymerisaten beschrieben wird, nicht überraschend. Es konnte jedoch nicht vorhergesehen werden, daß sich aus Copolymerisaten aus erfindungsgemäßen Monomeren, Acrylnitril und gegebenenfalls weiteren, mit Acrylnitril copolymerisierbaren Monomeren Membranen herstellen lassen, die mit Vorteil bei der Umkehrosmose und Ultrafiltration eingesetzt werden können.

Die Erfindung betrifft auch die Verwendung der Monomeren der Formel I zur Herstellung von neuen semipermeablen Membranen, die insbesondere zur Umkehrosmose und Ultrafiltration geeignet sind.

Umkehrosmose und Ultrafiltration sind Verfahren zur Stofftrennung. Die Stofftrennung erfolgt bei diesen Trennprozessen in der Weise, daß eine wässrige Lösung unter Druck über die Oberfläche einer semipermeablen Membran geleitet wird, wobei das Lösungsmittel und eventuell ein Teil der gelösten Stoffe die Membran passieren, während die übrigen Komponenten der Lösung an der Oberfläche der Membran zurückgehalten werden und in der Lösung aufkonzentriert werden können.

Technische Bedeutung haben hauptsächlich Membranen aus Cellulosederivaten, insbesondere

Celluloseacetat. Trotz ihrer guten Leistung hinsichtlich Wasserdurchlässigkeit und Trennvermögen weisen sie auch nachteilige Eigenschaften auf, die ihre allgemeine Verwendbarkeit einschränken. Es sind dies die mangelnde Chemikalienbeständigkeit, insbesondere die Hydrolyseempfindlichkeit bei hohem und niedrigem pH-Wert sowie die Anfälligkeit gegenüber einem Abbau durch Mikroorganismen. Dies führt im Laufe der Zeit zu einer Verschlechterung der Membraneigenschaften.

Es wurden bereits Membranen aus Polyacrylnitril für die umgekehrte Osmose hergestellt, die ein bestimmtes Salzrückhaltevermögen bei allerdings geringer Wasserpermeabilität (S.W. Saltonstall Jr. et al., OSW Res. Der. Progr. Report Nr. 220 (1966)) aufwiesen. In den deutschen Auslegeschriften 2 145 183 und 2 346 011 werden ionische Acrylnitrilcopolymerisate beschrieben, die zwar über eine hohe Wasserpermeabilität verfügen, jedoch nur zur Filtration von Lösungen makromolekularer Stoffe, d.h. zur Abtrennung von höhermolekularen Partikeln geeignet sind.

Es stehen daher noch keine Membranen mit einer hohen Wasserpermeabilität sowie einer guten Selektivität im Bereicht von Stoffen mit mittleren Molekulargewichten und ohne die vorstehend beschriebenen Nachteile der gebräuchlichen Celluloseacetatmembranen zur Verfügung.

Es wurde überraschenderweise gefunden, daß aus Maleinimidoaryl-aryl-disulfimid/Acrylnitril-Copolymerisaten Membranen hergestellt werden können, die die nachteiligen Eigenschaften der herkömmlichen Membranen auf der Basis von Celluloseacetat nicht besitzen und eine niedrige Trenngrenze von ca. 500 sowie eine Wasserpermeabilität aufweisen, die dennoch erheblich größer ist als beispielsweise die von aus reinem Polyacrylnitril hergestellten Membranen.

Gegenstand der Erfindung ist daher auch die Verwendung der Verbindungen der Formel I zur Herstellung von insbesondere zur Ultrafiltration und Umkehrosmose geeigneten semipermeablen Membranen, die aus Copolymerisaten von copolymerisierten Einheiten von

I) 50—99 Gew.-%, vorzugsweise 80—95 Gew.-% Acrylnitril,
II) 1—50 Gew.-%, vorzugsweise 5—20 Gew.-% mindestens eines Monomeren der allgemeinen Formel I

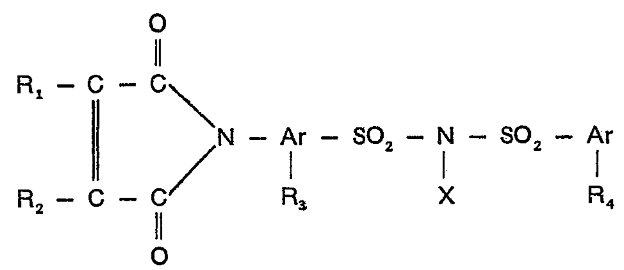

(I)

worin
Ar, $R_1$, $R_2$, $R_3$, $R_4$ und X die eingangs angegebene Bedeutung besitzen, und
III) 0—30 Gew.-%, vorzugsweise 0—15 Gew.-% mindestens eines weiteren olefinisch ungesättigten, mit Acrylnitril copolymerisierbaren Monomeren bestehen, wobei
die Summe der Prozentgehalte I bis III stets 100 beträgt.

Als weitere olefinisch ungesättigte, mit Acrylnitril copolymerisierbare Monomere III) seien Beispielsweise genannt:
(Meth)Acrylsäure, (Meth)Acrylamid, (Meth)Acrylsäurealkylester mit 1—12, vorzugsweise 1—4 C-Atomen in der Alkoholkomponente, Hydroxyalkyl-(meth)-acrylate mit 2—4 C-Atomen in der Alkylgruppe, Sulfoalkyl-(meth)-acrylate mit 2—4 C-Atomen in der Alkylgruppe, Maleinsäure, Vinylchlorid, Vinylidenchlorid—die vorstehenden Monomeren sind bevorzugt—ferner Butadien, Styrol, Fumarsäure, Maleinsäurehalbamid, das gegebenenfalls am Stickstoff durch $C_1$—$C_4$-Alkylgruppen mono- oder disubstituiert sein kann; Maleinimid, das gegebenenfalls durch $C_1$—$C_4$-Alkyl oder Phenyl am Stickstoff substituiert sein kann; Vinylpyrrolidon, Vinylpyridin oder Methacrylsäuresalicylat (Salicylsäure-O-methacrylat).

Die herstellung der Copolymerisate aus den Monomeren I bis III erfolgt nach bekannten Verfahren, beispielsweise nach dem Verfahren des deutschen Patents 1 089 548.

Die erhaltenen Copolymerisate enthalten die eingebauten Monomereinheiten im wesentlichen statistisch verteilt. Obwohl es prinzipiell möglich ist, die Molekulargeweichte der Polymerisate in weiteren Grenzen nach bekannten Methoden zu variieren, sind für die Herstellung von Membranen Polymerisate mit relativen Viskositäten $\eta_{rel}$ im Bereich von etwa 1,6 bis 4,6 besonders geeignet. Für die Herstellung der Membranen wird z.B. eine homogene Lösung des erhaltenen Copolymerisats aus den Monomeren I bis III (= Polymeres) in einem geeigneten Lösungsmittel vorzugsweise einem Lösungsmittel des Amid-Typs, hergestellt. 5—35 Gew.-% des Polymeren, bezogen auf die Gesamtmenge von Polymer und Lösungsmittel, werden unter Zusatz von 1—10 Gew.-% eines Alkali- oder Erdalkalisalzes, vorzugsweise LiCl, LiBr, $LiNO_3$, $MgCl_2$, $CaCl_2$, $CaBr_2$ oder eines organischen Amins wie Triäthylamin, Tripropylamin, Pyridin, Äthanolamin, Triäthanolamin in einem polaren aprotischen Lösungsmittel gelöst. Bevorzugte Lösungsmittel sind Dimethylformamid, Dimethylacetamid, N-Methylpyrrolidon, Hexamethylphosphorsäuretriamid, Dimethylsulfoxid bzw. deren Gemische Zur Beschleunigung des Lösungsvorganges kann gegebenenfalls erwärmt werden.

Diese Gießlösung wird zur Filmherstellung verwendet, indem man sie auf eine Glas- oder Metallunterlage oder irgendeine andere geeignete Unterlage wie z.B. auf ein fortlaufendes Band oder eine Trommel in einer Schichtdicke von 150—500 nm aufträgt.

Durch eine Wärmebehandlung erreicht man ein teilweises Abdampfen des Lösungsmittels. Die Trocknung des Films erfolgt bei einer Temperatur von 40—150°C über eine Zeitdauer von 2—60 Minuten. Dieser Schritt kann in Abhängigkeit von den gewünschten Membraneigenschaften des Films auch ausgelassen werden.

Nach einer Abkühlphase von einigen Minuten wird der Film in ein Fällungsbad getaucht und ca. 60 Minuten darin belassen. Als Flüssigkeit zur Ausfällung kommen solche Lösungsmittel in Betracht, die mit dem organischen Lösungsmittel der Gießlösung mischbar sind und gegebenenfalls das Salz zu lösen vermögen, aber für das Polyamid ein Nichtlösungsmittel sind. Hierfür geeignet sind Wasser, Methanol, Äthanol, i-Propanol, gegebenenfalls unter Zusatz von Salzen wie z.B. $CaCl_2$. Bevorzugtes Fällungsmittel ist Wasser. Die Temperatur des Fällungsbades kann zwischen 0° und 50° liegen, bevorzugt zwischen 0° und 25°C.

Die Membranen können in Form von Folien, Röhren, Schläuchen oder Hohlfasern verwendet werden. Die Herstellungstechniken für Schläuche, Röhren und Hohlfasern entsprechen sinngemäß dem oben geschilderten Verfahren. Es werden in diesem Fall die zur Herstellung von Röhren, Schläuchen und Hohlfasern aus Polymerisatlösungen dem Fachmann bekannten Verfahren benutzt.

Die Membranen können zur Anreicherung und Rückgewinnung verwertbarer Stoffe und zur Entfernung von unerwünschten Stoffen eingesetzt werden, so z.B. bei der Behandlung von Abwässern in der Farbstoff-, Papier- und Textilindustrie.

Die Membranen sind zweckmäßig asymmetrische Membranen, die durch folgende Struktur charakterisiert sind: Die eigentliche selektive Trennschicht ist äußerst dünn und geht annähernd kontinuierlich in eine Unterschicht mit Porenstruktur aus demselben Material über, die als Träger- oder Stützschicht dient. Es ist ein Vorzug solcher Membranen, daß alle Stoffe an der Oberfläche der Membran abgetrennt werden, wo sie durch die Strömung der Beschickungslösung entfernt werden können. Auf diese Weise wird die Lebensdauer der Membranen erhöht, da sie nicht so schnell verstopfen können.

Zur Bestimmung der Membraneigenschaften wird die fertige Membran auf eine poröse Sinterplatte aus Metall, auf der ein Filterpapier liegt, aufgebracht, und in eine Druckfiltrationsapparatur eingesetzt, in der die verschiedenen Lösungen der Testsubstanzen in Wasser bei Raumtemperatur und unter verschiedenen Drucken an der Membranoberfläche vorbeigepumpt werden. Die Pumpleistung beträgt 21,5 l/h, die wirksame Membranfläche ca. 40 $cm^2$.

Der Durchsatz an Wasser, angegeben in Liter/$m^2$ Tag (=l/$m^2$d), gibt die Filtrationsleistung der Membran an. Die prozentuale Zurückhaltung wird üblicherweise wie folgt angegeben:

$$\text{Zurückhaltung} = 1 - \frac{\text{Konzentration des Filtrats an gelöstem Stoff}}{\text{Konzentration der Ausgangslösung an gelöstem Stoff}} \, . \, 100$$

Die Membranen besitzen einerseits eine hohe Wasserpermeabilität, sind aber andererseits in der Lage, Stoffe mit einem Molekulargewicht von 500 von solchen mit Molekulargewichten um 100 zu trennen, wobei als Maßstab für die Zurückhaltung bzw. Abtrennung das Molekulargewicht des oder der gelösten Komponenten betrachtet werden soll.

Diese Membranen werden zur Konzentrierung, Entfernung oder Gewinnung der verschiedensten Stoffe aus wäßrigen Lösungen durch Umkehrosmose, Ultrafiltration oder ähnliche Verfahren verwendet. Die vorliegende Erfindung betrifft ebenfalls die Verwendung der Membranen.

Die in den Beispielen angegebenen Prozentgehalte beziehen sich auf das Gewicht, sofern nicht anders vermerkt.

Beispiel 1

2,2 kg 3-Amino-N-(phenylsulfonyl)-benzolsulfonamid und 2,8 kg Maleinsäureanhydrid werden auf ca. 110—120°C erwärmt und mit 2 ml Triäthylamin als Katalysator versetzt. Nach ca. 2 Stunden wird die Temperatur langsam auf 150°C gesteigert und überschüssiges Maleinsäureanhydrid unter Vakuum abdestilliert, anschließend auf 110°C abgekühlt (eine isolierte Probe des 3-Maleinsäuremonoamidobenzol-benzol-disulfimids schmilzt bei 185°C) und mit 4,5 kg Essigsäureanhydrid und 490 g Natriumacetat (wasserfrei) versetzt. Nach ca. 40 Min. bei 100°C wird bei 20 Torr Essigsäure bzw. Anhydrid abdestilliert, wobei das Rohprodukt auskristallisiert. Abschließend wird mit ca. 5 l Isopropanol behandelt und abgesaugt. Die Ausbeute an 3-Maleinimidobenzol-benzoldisulfimid beträgt 2,3 kg entsprechend 84% der Theorie, Fp 157—160°C. IR: $V_{CO}$ = 1720 (s)/1785 (w) $cm^{-1}$.

Die Ausbeuten in den Beispielen 1 und 2 sind auf eingesetztes 3-Amino-N-(phenylsulfonyl)-benzolsulfamid bezogen.

Beispiel 2

40 g 3-Amino-N-(phenylsulfonyl)-benzolsulfonamid und 25 g Methylmaleinsäureanhydrid werden auf 120 bis 130°C erwärmt und mit geringer Menge Triäthylamin als Katalysator versetzt. Nach ca. 2 Stunden wird die Temperatur auf 175°C gesteigert und überschüssiges Methylmaleinsäureanhydrid im Vakuum abdestilliert. Nach beendeter Destillation(eine isolierte Probe des 3-Methylmaelinsäuremonoamidobenzol-benzol-disulfimids schmilzt bei 165°C) werden bei ca. 90°C 94 g Essigsäureanhydrid und 9 g Natriumacetat zugegeben. Nach ca. 30 Minuten wird abgekühlt, das feste Produkt abgesaugt und mit Isopropanol gewaschen. Ausbeute an 3-Methylmaleinimidobenzol-benzol-disulfimid: 44 g, entsprechend 90% der Theorie, Fp (Isopropanol) = 171°C IR: $V_{CO}$ = 1720 (s); 1775 (w) $cm^{-1}$

Beispiel 3

5 g 3-Maleinimidobenzol-benzol-disulfimid nach Beispiel 1 und 20 g Methacrylsäure werden in 30 ml Methanol gelöst und nach Zugabe von 0,5 g Azodiisobutyronitril unter Rückfluß erwärmt. Nach 80 Minuten wird die Polymerisation abgebrochen und die viskose Lösung in Aceton getropft, wobei ein Polymerprodukt ausfällt. Die Reinigung des Produktes erfolgt durch erneutes Auflösen in Methanol und Ausfällen aus Aceton. Es wird ein Copolymerisat erhalten, das aus 15 Gew.-% 3-Maleinimidobenzol-benzol-disulfimid- und 85 Gew.-% Methacrylsäureeinheiten besteht. Die Einbauraten wurden stets aus den S-Analysendaten ermittelt.

Beispiel 4

5 g 3-Maleinimidobenzol-benzol-disulfimid und 10 g Methacrylsäure werden in gleicher Weise wie in Beispiel 3 copolymerisiert. Das Copolymerisat enthält 24 Gew.-% 3-Maleinimidobenzol-benzoldisulfonimid und 76 Gew.-% Methacrylsäureeinheiten.

Beispiel 5

*A.) Copolymerisation des 3-Maleinimidobenzol-benzol-disulfimid (Na-Salz) mit Acrylnitril*

In 760 ml Wasser werden 53 g Acrylnitril und 5,9 g einer Verbindung der Formel

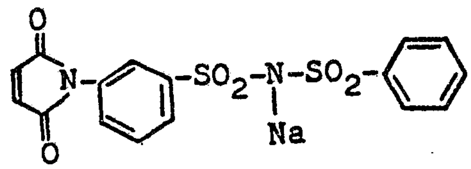

auf 60°C erwärmt. Dann wird der pH-Wert mit verdünnter Schwefelsäure auf 3 eingestellt und die Polymerisation durch Zugabe von 0,7 g Kaliumperoxodisulfat und 2,9 g Natriumsulfit gestartet. nach 5 h wird das ausgefallene Copolymerisat abgesaugt, neutral gewaschen und getrocknet. Das Copolymerisat enthält ca. 94.6 Gew.-% Acrylnitrileinheiten und 5.4 Gew.-% eingebaute Einheiten des 3-Maleinimidobenzol-benzol-disulfimids (Na-Salz), ermittelt durch Schwefelanalyse.

$\eta_{rel}$: 2,70 (0,5 g/100 ml in N-Methylpyrrolidon)

Das Natriumsalz des Maleinimidobenzol-benzol-disulfimids wird aus der wäßrigen Lösung des Disulfimids durch Zugabe von wäßrigem Natriumhydroxid, Einengen der wäßrigen Lösung und durch anschließendes Auskristallisieren erhalten.

*B) Membranherstellung*

Aus 15 g des vorstehend beschriebenen Polymeren, 4,5 g $CaCl_2$ und 130,5 g N-Methylpyrrolidon wurde unter Rühren und Erwärmen auf 60°C eine Lösung hergestellt. Nach Filtrieren und Entfernen restlicher Lufblasen erhielt man eine gebrauchsfertige Gießlösung.

Auf eine Glasplatte wurde ein Film von 250 nm Dicke aufgebracht und anschließend 20 min bei 50°C auf einer Heizplatte erhitzt. Nach einer Abkühlphase von 10 min wurde der Film in ein Eis/Wasser bad getaucht und 30 min darin belassen. Während dieser Zeit löste sich der Film von der Glasplatte. Die fertige Membran wurde in Wasser bei Raumtemperatur aufbewahrt.

Die Membran ergab bei der Prüfung die in der Tabelle aufgeführten Ergebnisse:

| Substanz | Konzentration % | Zurückhaltung % | Durchfluß $1/m^2d$ |
|---|---|---|---|
| NaCl | 1 | 0 | — |
| Natriumlaurylsulfat | 1 | 73 | 3100 |
| Kongorot | 0,1 | 99,9 | 3400 |

Druck: 20 bar

Beispiel 6

*A.) Copolymerisation des 3-Maleinimidobenzol-benzol-disulfimids mit Acrylnitril*

53 g Acrylnitril und 13,25 g 3-Maleinimidobenzol-benzol-disulfimid werden in 760 ml Wasser gelöst. Der pH-Wert wird mit verdünnter Schwefelsäure auf 3 eingestellt und bei 60° die Polymerisation durch Zugabe von 0,7 g Kaliumperoxodisulfat und 2,9 g Natriumsulfit gestartet. Das ausgefallene Polymerisat wird nach 5 h abgesaugt, gewaschen und getrocknet. Es enthält etwa 91.8 Gew.-% eingebaute Acrylnitrileinheiten und 8.2 Gew.-% eingebaute Einheiten des 3-Maleinimidobenzol-benzol-disulfimids

$\eta_{rel}$: 2,65 (0,5 g/100 ml in N-Methylpyrrolidon)

*B.) Membranherstellung*

Zur Herstellung einer Gießlösung wurden 10 g des vorstehend beschriebenen Polymeren, 3 g $CaCl_2$ und 87 g N-Methylpyrrolidon verwendet. Die Gießlösung wurde zu einem Film von 250 nm Dicke vergossen, der 20 min bei 60°C getrocknet wurde.

Die Membran zeigte eine Zurückhaltung von 99,9% (Kongorot, 0,1%) und eine Wasserpermeabilität von 2100 $l/m^2d$ bei einem Druck von 20 bar.

Beispiel 7

*A.) Copolymerisation des 3-Maleinimidobenzol-benzol-disulfimids mit Acrylnitril und Methylmethacrylat*

Unter Stickstoff werden bei 60° 53 g Acrylnitril, 3,12 g Acrylsäuremethylester und 6,25 g 3-Maleinimidobenzol-benzol-disulfimid in 780 ml Wasser gelöst und der pH-Wert mit verdünnter Schwefelsäure auf 3 eingestellt. Nach Zugabe von 0,7 g Kaliumperoxodisulfat und 2,9 g Natriumsulfit wird 5 Stunden bei 60° nachgerührt, abgesaugt, neutral gewaschen und getrocknet.

$\eta_{rel}$: 1,98 (0,5 g/100 ml in N-Methylpyrrolidon)

*B.) Membranherstellung*

20 g des vorstehend beschriebenen Polymeren und 6 g $CaCl_2$ wurden in 174 g N-Methylpyrrolidon gelöst. Aus dieser Gießlösung wurde ein 250 nm dicker Film hergestellt, der 10 min bei 60°C getrocknet wurde.

Bei 20 bar wies die Membran einen Durchfluß von 3500 $l/m^2d$ und eine 99,9% Zurückhaltung gegenüber Kongorot (0,1%) auf.

*Vergleichsbeispiel*

12 g eines Homopolymerisats des Acrylnitrils werden unter Zusatz von 3,6 g $CaCl_2$ in 84,4 g N-Methylpyrrolidon in Lösung gebracht. Aus dieser Gießlösung wurde ein Film mit 250 nm Dicke auf einer Glasplatte hergestellt und anschließend das Lösungsmittel 20 Min. bei 70°C verdampft und der Film in ein Fällungsbad (Eis/Wasser) eingetaucht. Es wurde bei 20 bar ein Wasserdurchfluß von nur 1100 $l/m^2d$ gemessen, die Entfernung von Kongorot betrug 99,9%.

**Patentansprüche**

1. Verbindungen der allgemeinen Formel (I)

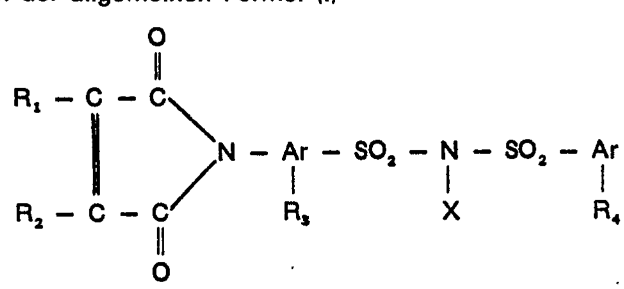

(I)

in der

Ar einen Benzol- oder Naphthalinrest;

$R_1$ und $R_2$ gleich oder verschieden voneinander H oder $CH_3$;

$R_3$ und $R_4$ gleich oder verschieden voneinander H, Phenyl, Alkyl verzweigt oder linear mit 1—6 C-Atomen, Hydroxyalkyl verzweigt oder linear mit 1—6 C-Atomen oder Cl, F und

X, H, Na, K und $NH_4$ darstellen.

2. 3-Maleinimidobenzol -benzol-disulfimid.

3. 3-Methylmaleinimidobenzol-benzol-disulfimid.

4. Verfahren zur Herstellung der N-substituierten Maleinimidderivate nach Anspruch 1 durch Umsetzung von Maleinsäureanhydrid oder einem durch $CH_3$, substituierten Maleinsäureanhydrid mit einem entsprechenden Aminoaryl-aryl-disulfimid und anschließender Cyclodehydratisierung der gebildeten N-Substituierten Maleinamidsäure in einem Anhydrid einer gesättigten Fettsäure mit maximal 5 C-Atomen in Gegenwart eines Katalysators, dadurch gekennzeichnet, daß die Umsetzung der Maleinsäureanhydrids oder methylsubstituierten Maleinsäureanhydrids mit dem Aminoaryl-aryl-disulfimid in der Schmelze in Abwesenheit oder in Gegenwart eines Katalysators erfolgt und das Maleinsäureanhydrid gegenüber dem Aminoaryl-aryl-disulfimid in einem 1 bis 5fachen molaren Überschuß eingesetzt wird.

5. Verwendung der Verbindungen nach Anspruch 1 bis 3 zur Herstellung von insbesondere zur Ultrafiltration und Umkehrosmose geeigneten semipermeablen Membranen, die aus Copolymerisaten von copolymerisierten Einheiten von

I) 50—99 Gew.-% Acrylnitril

II) 1—50 Gew.-% mindestens eines Monomeren der allgemeinen Formel I

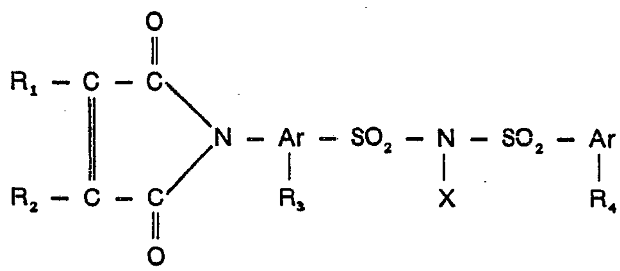

in der

Ar, $R_1$, $R_2$, $R_3$ $R_4$ und X die in Anspruch 1 angegebene Bedeutung besitzen,

und

III) 0—30 Gew.-% mindestens eines weiteren olefinisch ungesättigten, mit Acrylnitril copolymerisierbaren Monomeren bestehen, wobei die Summe der Prozentgehalte I) bis III) stets 100 beträgt.

**Revendications**

1. Composés de formule générale (I):

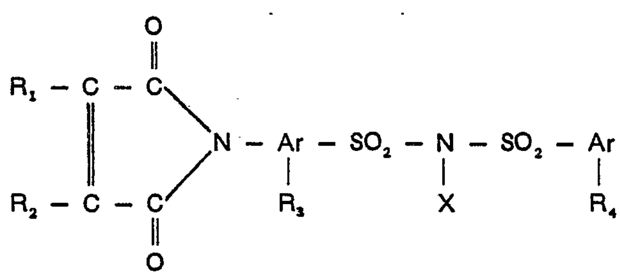

(I)

dans laquelle:

Ar est un reste de benzène ou de naphtalène;

$R_1$ et $R_2$ égaux ou différents l'un de l'autre, représentent H ou $CH_3$;

$R_3$ et $R_4$ égaux ou différents l'un de l'autre, représentent H, un groupe phényle, un groupe alkyle ramifié ou linéaire ayant 1 à 6 atomes de carbone, un groupe hydroxyalkyle ramifié ou linéaire ayant 1 à 6 atomes de carbone ou Cl, F et

X représente H, Na, K ou $NH_4$.

2. Le 3-maléimidobenzène-benzène-disulfimide.

3. Le 3-méthylmaléimidobenzène-benzène-disulfimide.

4. Procédé de production des dérivés de maléimide substitués sur l'atome d'azote suivant la

revendication 1 par réaction d'anhydride d'acide maléique ou d'un anhydride d'acide maléique substitué par $CH_3$ avec un amino-aryl-aryl-disulfimide correspondant et cyclodéshydratation subséquente de l'amido-acide maléique substitué sur l'atome d'azote ainsi formé dans un anhydride d'un acide gras saturé ayant au maximum 5 atomes de carbone, en présence d'un catalyseur, caractérisé en ce que la réaction de l'anhydride d'acide maléique ou de l'anhydride d'acide maléique substitué avec l'amino-aryl-aryl-disulfimide est conduite à l'état fondu en l'absence ou en présence d'un catalyseur et l'anhydride d'acide maléique est utilisé, par rapport à l'amino-aryl-aryl-disulfimide, en un excès molaire d'un facteur 1 à 5.

5. Utilisation des composés suivant les revendications 1 à 3 pour la production de membranes semiperméables convenant notamment à l'ultrafiltration et à l'osmose, inverse, qui sont constituées par des copolymérisats de motifs copolymérisés de:

(I) 50 à 99% en poids d'acrylonitrile,
(II) 1 à 50% en poids d'au moins un monomère de formule générale (I)

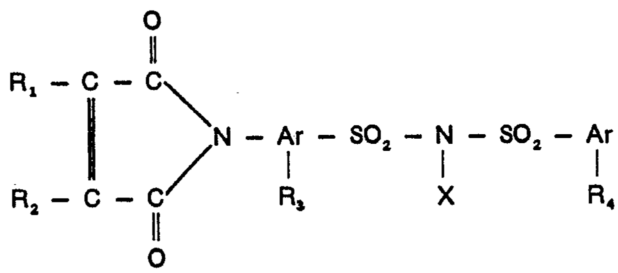

dans laquelle
Ar, $R_1$, $R_2$, $R_3$, $R_4$ et X ont la définition donnée dans la revendication 1, et
(III) 0 à 30% en poids d'au moins un autre monomère à insaturation oléfinique, copolymérisable avec l'acrylonitrile,
la somme des pourcentages de (I) à (III) s'élevant toujours à 100.

**Claims**

1. Compounds of the general formula (I):

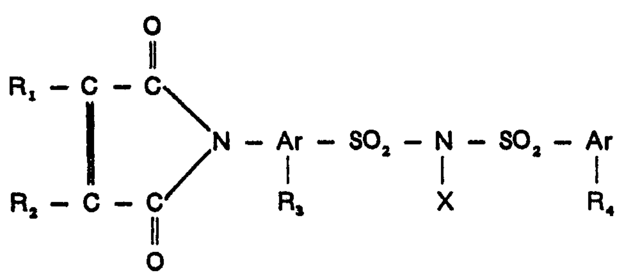
(I)

in which
Ar denotes a benzene or naphthalene group;
$R_1$ and $R_2$ are identical or different and denote H or $CH_3$;
$R_3$ and $R_4$ are identical or different and denote H, phenyl, branched-chain or linear alkyl having from 1—6 C-atoms, branched-chain or linear hydroxyalkyl having from 1—6 C-atoms, or Cl, or F and X denotes H, Na, K and $NH_4$.

2. 3-maleic-imidobenzene-benzene-disulphimide.

3. 3-methyl maleic-imidobenzene-benzene-disulphimide.

4. A process for the preparation of the N-substituted maleic imide derivatives according to Claim 1, by reacting maleic acid anhydride, or a maleic acid anhydride which is substituted by $CH_3$, with a corresponding aminoaryl-aryl-disulphimide and subsequently amido acid in an anhydride of a saturated fatty acid having a maximum of 5 C-atoms in the presence of a catalyst, characterised in that the reaction of the maleic acid anhydride or substituted maleic acid anhydride with the aminoaryl-aryl-disulphimide is carried out in the melt and with or without a catalyst and the maleic acid anhydride is used in a 1 to 5 times molar excess over the aminoaryl-aryl-disulphimide.

5. Use of the compounds according to claim 1 to 3 for the production of semipermeable membranes suitable in particular for ultrafiltration and reverse osmosis, which semipermeable membranes consist of copolymers of copolymerised units of

I) 50 to 99% by weight of acrylonitrile,

II) 1 to 50% by weight of at least one monomer of the general formula (I)

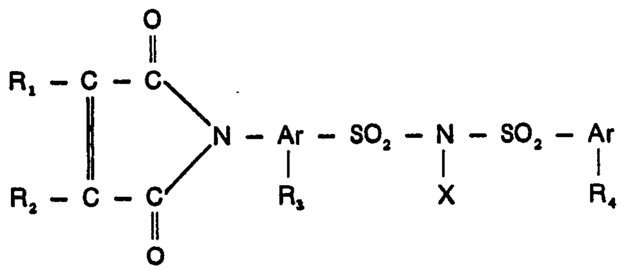

in which
Ar, $R_1$, $R_2$, $R_3$ $R_4$ and X have the meaning given in Claim 1, and
III) 0—30% by weight of at least one other olefinically unsaturated monomer which is copolymerisable with acrylonitrile,
the sum of the percentage contents of (I) to (III) being in all cases 100.